# EUROPÄISCHE PATENTANMELDUNG

(11) **EP 2 226 076 A1**
(43) Veröffentlichungstag der Anmeldung: **08.09.2010**
(21) Anmeldenummer: 09153622.7
(22) Anmeldetag: 25.02.2009
(51) Int. Cl.: A61K 36/31, A61P 3/10

(54) **Pflanzlicher Extrakt zur Prophylaxe und Behandlung von hyperglykämischen Erkrankungen**

(71) Anmelder: Vollert, Henning, 23795 Bad Segeberg (DE)
(72) Erfinder: Vollert, Henning, 23795 Bad Segeberg (DE)
(74) Vertreter: Guder, André

(57) **Zusammenfassung**

Die vorliegende Erfindung betrifft einen Extrakt aus einer Pflanze der Gattung Brassica zur Behandlung und/oder Prophylaxe einer hyperglykämischen Erkrankung. Die Erfindung betrifft ferner die Verwendung eines solchen Extrakts zur Herstellung einer pharmazeutischen Zusammensetzung, eines diätetischen Lebensmittels und/oder eines Nahrungsergänzungsmittels zur Behandlung und/oder Prophylaxe einer hyperglykämischen Erkrankung, wie Fettsucht, Diabetes oder einer von Diabetes verursachten Folgeerkrankung. Die Erfindung betrifft ferner eine pharmazeutische Zusammensetzung, ein diätetisches Lebensmittel und ein Nahrungsergänzungsmittel, die einen solchen Extrakt umfassen.

## Beschreibung

Die vorliegende Erfindung betrifft einen Extrakt aus einer Pflanze der Gattung Brassica zur Behandlung und/oder Prophylaxe einer hyperglykämischen Erkrankung. Die Erfindung betrifft ferner die Verwendung eines solchen Extrakts zur Herstellung einer pharmazeutischen Zusammensetzung, eines diätetischen Lebensmittels und/oder eines Nahrungsergänzungsmittels zur Behandlung und/oder Prophylaxe einer hyperglykämischen Erkrankung, wie Fettsucht, Diabetes oder einer von Diabetes verursachten Folgeerkrankung. Die Erfindung betrifft ferner eine pharmazeutische Zusammensetzung, ein diätetisches Lebensmittel und ein Nahrungsergänzungsmittel, die einen solchen Extrakt umfassen.

### GEBIET DER ERFINDUNG

Die Zahl der Diabetes-Toten ist nach Angaben des Statistischen Bundesamts seit 1980 um 29 Prozent gestiegen. Diabetes mellitus ist damit verantwortlich für knapp drei Prozent aller Sterbefälle (1980: zwei Prozent) in Deutschland. Parallel dazu steigt die Zahl der übergewichtigen Menschen in Deutschland stark an; jeder zweite Deutsche ist bereits übergewichtig, jeder fünfte sogar fettleibig. Bei diesen Menschen ist das Risiko, an Diabetes zu erkranken, dramatisch erhöht.

Aktuelle Hochrechnungen gehen davon aus, dass bis zu 7 % der Deutschen (Hauner H. Dtsch Med Wochenschr 2005; 130 Suppl 2:S.64-65), d.h. rund 6 Millionen Menschen, wegen eines Diabetes in Behandlung sind. Die Anzahl der Neuerkrankungen nimmt dabei mit steigendem Alter zu; in der Altersgruppe der über 60-jährigen liegt der Anteil der an Diabetes Erkrankten bei 18-28 %. Parallel dazu sinkt das Manifestationsalter betroffener Typ-2-Diabetiker stetig. Typ-2-Diabetes ist mit rund 11 % die vierthäufigste Diagnose der hausärztlichen Internisten und mit rund 8 % die fünfthäufigste Diagnose aller Allgemeinärzte (Wittchen HU, (HYDRA)-Studie, Fortschr Med Orig 2003; 121). Aktuelle Schätzungen der WHO gehen davon aus, dass sich die Zahl der an Diabetes Erkrankten bis zum Jahr 2025 verdoppeln wird (World Health Organization, Diabetes mellitus, Fact sheet No. 138, 2002).

Mit der Nahrung wird eine Vielzahl an Fetten, Kohlehydraten und Proteinen aufgenommen, die den menschlichen Körper mit der benötigten Energie versorgen. Eine zu starke Aufnahme und insbesondere eine zu schnelle Aufnahme von Zuckern stellt eine starke Belastung des Stoffwechsels dar und gilt als ein wesentlicher Risikofaktor für Diabetes, Übergewicht und Fettleibigkeit. Es besteht daher ein anhaltender Bedarf an neuen Mitteln für die Behandlung von hyperglykämischen Erkrankungen wie Diabetes und Fettleibigkeit.

Zahlreiche Verbindungsklassen sind für die Behandlung des Diabetes mellitus, insbesondere des Diabetes mellitus Typ II, vorgeschlagen worden. In verschiedenen Internationalen Anmeldungen sind beispielsweise Substanzen vorgeschlagen worden, die den Natrium-abhängigen Glucose-Transporter 2 (SGLT-2) hemmen (siehe z.B. WO 98/31697, WO 02/083066, WO 03/099836 und WO 01/31697). Dieses Transportprotein resorbiert im proximalen Tubulus der Niere Glucose und Natrium aus dem Primärharn. Spezifische SGLT-2-Inhibitoren sind in der Lage, die Aufnahme von Glucose in den Nierentubuli zu verhindern, was zur Wiederherstellung von normalen Plasmaglucosespiegeln führt. Eine langfristige Behandlung mit SGLT-2-Inhibitoren von mehr als 6 Monaten hat im Tierversuch zu einer verstärkten Insulinempfindlichkeit, einer verbesserten Insulin-Reaktion und zu einer verzögerten Ausbildung von Diabetes-bedingten Komplikationen geführt. Die Funktion des Transporters SGLT-2 beruht auf der verstärkten konzentrationsabhängigen Ausscheidung von Glucose, die zu einer Senkung des Blutzuckers im Körper führt. Dabei wird jedoch keine Hypoglykämie verursacht. Der Mechanismus der Blutzuckersenkung ist unabhängig von einer ggf. bestehenden Insulinresistenz oder einer mangelnden Insulinproduktion durch die Betazellen der Bauchspeicheldrüse. Neben SGLT-2 gibt es einen weiteren Natrium-abhängigen Glucose-Transporter, SGLT-1, der ebenfalls mit der Behandlung des Diabetes in Verbindung gebracht wird. SGLT-1 wird im Dünndarm sowie im S3-Segment des proximalen Nierentubulus gefunden. Er ist für die aktive Aufnahme von Glucose verantwortlich. Im Vergleich zu SGLT-2 weist SGLT-1 für einige Zucker eine veränderte Substratspezifität auf.

Verschiedene Präparate, die zu einer Hemmung von SGLT-1 und/oder SGLT-2 führen, befinden sich derzeit in klinischen Erprobungen. Im Rahmen der vorliegenden Erfindung wurde überraschenderweise festgestellt, dass ein Extrakt aus einer Pflanze der Gattung Brassica zu einer Hemmung des Natrium-abhängigen Glucose-Transporters SGLT-1 führt. Demgemäß kommt ein solcher Extrakt bei der Behandlung und/oder bei der Prophylaxe einer hyperglykämischen Stoffwechselerkrankung wie Diabetes in Betracht. Die Extrakte führen über die Hemmung des Natrium-abhängigen Glucose-Transporters zur Wiederherstellung von normalen Glucosespiegeln im Plasma von Patienten, die an einer hyperglykämischen Erkrankung wie Diabetes leiden.

Demgemäß betrifft die Erfindung in einem ersten Aspekt einen Extrakt aus einer Pflanze der Gattung Brassica zur Behandlung und/oder Prophylaxe einer hyperglykämischen Erkrankung. Erkrankungen, die sich mit Hilfe der erfindungsgemäß vorgeschlagenen Brassica-Extrakte behandeln lassen, umfassen alle Krankheiten, die durch eine Hyperglykämie, d.h. durch einen erhöhten Blutzuckerspiegel gekennzeichnet sind, der den physiologisch normalen Wert von 140 mg/dl (7,8 mmol/l) übersteigt. Solche Erkrankungen umfassen insbesondere Fettsucht, Diabetes oder von Diabetes verursachte Folgeerkrankungen (wie z.B. Retinopathie, Neurophathie, Nephropathie und gestörte Wundheilung). Bei dem Diabetes, der mittels der Brassica-Extrakte behandelt werden soll, kann es sich um Diabetes Typ I oder Typ II handeln. Vorzugsweise handelt es sich um Diabetes Typ II. Auch eine wirksame Eindämmung von Übergewicht läßt sich mit Hilfe der erfindungsgemäßen Extrakte erreichen.

Die erfindungsgemäß zur Behandlung oder Prophylaxe vorgeschlagenen Extrakte können auch in vorteilhafter Weise mit anderen Wirkstoffen verwendet werden, z.B. zusammen mit anderen im Stand der Technik beschriebenen antidiabetischen Mitteln, wie z.B. Biguanide, Sulfonylharnstoffe, Glucosidaseinhibitoren, Thiazolidindione, Dipeptidylpeptidase IV (DP4)-Inhibitoren, Meglitinide, Glucagon-ähnliches Peptid-1 (GLP-1), PTP1B-Inhibitoren, Glycogenphosphorylase-Inhibitoren und Glucose-6-phosphatase-Inhibitoren.

Die im Rahmen der Erfindung vorgeschlagenen Extrakte können aus Spezies der Gattung Brassica gewonnen werden. Bei der Gattung Brassica handelt es sich um eine pflanzliche Gattung in der Familie der Kreuzblütengewächse (Brassicaceae). Besonders bevorzugt ist es, dass die Extrakte ausgehend von Pflanzen der Spezies Brassica oleracea hergestellt werden. Bei der Spezies Brassica oleracea handelt es sich um eine formenreiche Pflanzenart innerhalb der Gattung Brassica. Die Art umfasst zahlreiche Sorten, zu den u.a. Brokkoli (Brassica oleracea var. silvestris L.), Rotkohl (Brassica oleracea var. capitata f. rubra L.), Weißkohl (Brassica oleracea var. capitata f. alba), Wirsing (Brassica oleracea L. convar. capitata (L.) Alef. var. sabauda L.), Chinakohl (Brassica rapa ssp. pekinensis) und Grünkohl (Brassica oleracea convar. acephala var. sabellica) zählen. Besonders bevorzugt ist es, dass die Extrakte ausgehend von Grünkohl hergestellt werden. Neben Pflanzen der Spezies Brassica oleracea können jedoch auch andere Spezies der Gattung Brassica Verwendung finden.

Wie vorliegend verwendet bezeichnet der Begriff "Brassica-Extrakt" ein Stoffgemisch, das durch Aufschluß von Zellen einer Pflanze der Gattung Brassica und Gewinnung des Zellsafts erhalten wird. Die erfindungsgemäß vorgeschlagenen Brassica-Extrakte lassen sich durch eine Vielzahl von im Stand der Technik bekannten Verfahren herstellen.

In einer Ausführungsform der Erfindung wird zunächst Material einer Brassica-Pflanze bereitgestellt. Als Ausgangsmaterial für das erfindungsgemäße Verfahren eignen sich Brassica-Pflanzen in sämtlichen Entwicklungsstadien, vom Samen bis zur reifen Pflanze. Das pflanzliche Material wird in der Regel zunächst zerkleinert. Das Zerkleinern kann durch einfaches Zerstückeln der jeweiligen Pflanzenteile mit herkömmlichen Cuttern erreicht werden. Es lassen sich grundsätzlich alle Pflanzenteile als Ausgangspunkt für die Extraktbildung verwenden. Besonders gute Ergebnisse werden jedoch bei Verwendung von Blättern erzielt. Daher wird der Brassica-Extrakt gemäß einer bevorzugten Ausführungsform der Erfindung aus den Blättern der jeweiligen Brassica-Pflanze hergestellt. Vor der Zerkleinerung kann das pflanzliche Material bei Temperaturen zwischen 45°C und 100°C blanchiert werden, um bakterielle Verunreinigungen zu beseitigen und die Qualität des Aufschlusses zu verbessern. Des Weiteren wird durch das Blanchieren die Aktivität von Enzymen wie Hydrolasen, Lipasen und Oxidasen reduziert und somit die Stabilität und Qualität des Pflanzenmaterials erhöht.

In einem nächsten Schritt wird das pflanzliche Material aufgeschlossen, d.h. die zellulären Strukturen des Materials werden zerstört, sodass der Inhalt der Zellen freigesetzt wird. Der Aufschluß kann durch herkömmliche Verfahren zum Aufschluß pflanzlicher Zellen erreicht werden, beispielsweise durch wiederholtes Einfrieren und Auftauen, oder durch geeignete Apparaturen, wie z.B. durch Homogenisatoren, Hochdruckhomogenisatoren oder Ultraschallhomogenisatoren. Auch Kolloidmühlen oder French-Pressen können für den Aufschluß verwendet werden.

Darüber hinaus kann der Zellaufschluß auch enzymatisch erzielt werden. Zu diesem Zweck wird das pflanzliche Material mit geeigneten Enzymen versetzt, die zu einer Zerstörung der strukturellen Bestandteile der Zellen führen. Es hat sich gezeigt, dass eine Inkubation des pflanzlichen Materials mit Pektinase, Kollagenase und/oder Cellulase besonders geeignet ist, um die Zellen wirkungsvoll aufzuschließen. Die Inkubationszeit kann zwischen 30 Minuten und 24 Stunden liegen, vorzugsweise zwischen 6 Stunden und 4 Stunden, z.B. 2 Stunden. Die Temperatur kann im Bereich von 20°C und 60°C liegen, vorzugsweise zwischen 25°C und 35°C und sie sollte vorzugsweise im Bereich des Temperaturoptimums des jeweils verwendeten Enzyms liegen. Ein geeigneter pH-Wert des Reaktionsansatzes kann unter Verwendung geeigneter Puffer, wie z.B. Phosphat-, Carbonat-, Natriumhydrogencarbonatpuffer und/oder geeigneter Säuren, wie z.B. Citronensäure, Milchsäure oder Ascorbinsäure eingestellt werden.

Nach Aufschluß der Zellen, kann der erhaltende Zellsaft direkt zur Hemmung des Natrium-abhängigen Glucose-Transporters 1 (SGLT-1) verwendet werden. Es ist allerdings bevorzugt, den nach dem Aufschluß der Zellen erhaltenen Pflanzensaft weiteren Reinigungsschritten zu unterziehen.

Beispielsweise kann das aus dem Zellaufschluß erhaltene Material einer Trocknung oder Gefriertrocknung mit anschließender Lösungsmittel-Extraktion unterzogen werden. Hier kann beispielsweise zunächst eine Sprühtrocknung eingesetzt werden. Das getrocknete oder gefriergetrocknete pflanzliche Material kann anschließend mit einem wässrigen oder organischen Lösungsmittel extrahiert werden. Dies kann mit einem herkömmlichen Extraktionsmittel durchgeführt werden, z.B. mit Ethanol, Ethylacetat oder mit anderen organischen Lösungsmitteln. Auch eine Extraktion mit Gasen, wie z.B. mit Stickstoff ist möglich. Bei Verwendung eines flüssigen Lösungsmittels beträgt die Inkubationszeit 0,5 bis 24 Stunden, vorzugsweise 2 bis 8 Stunden. Bei Verwendung von Stickstoff erfolgt die Extraktion bei Temperaturen zwischen 4°C und 37°C in einem Zeitraum von 0,25 bis 3 Stunden, vorzugsweise in einem Zeitraum von 20 und 60 Minuten.

Alternativ zu einer Trocknung/Extraktion kann der nach Aufschluß der Zellen erhaltene Pflanzensaft auch einer chromatographischen Reinigung unterzogen werden, beispielsweise unter Verwendung einer Ionenaustauschchromatographie oder einer Affinitätschromatographie. Die mit Ethanol oder Puffer eluierten Fraktionen können direkt verwendet oder eingetrocknet werden.

Es wird angenommen, dass die den SGLT-1 hemmenden Bestandteile des Extrakts zur Gruppe der Flavonoide und/oder Glycoside gehören. Flavonoide zählen zur Gruppe der sekundären Pflanzenstoffe, und sie stellen eine umfangreiche Stoffklasse polyphenolischer Verbindungen dar, die in Lebensmitteln pflanzlicher Herkunft verbreitet vorkommen. Glycoside sind chemische Verbindungen, bei denen ein Alkohol (R-OH) über eine glykosidische Bindung an einen Zuckerteil gebunden ist. Es handelt sich daher bei einem Glycosid um das Vollacetal eines Zuckers. Beispiele für typische Glycoside, die als sekundäre Pflanzenmetabolite vorkommen, sind die Glucosinolate. Grünkohl und andere Sorten der Pflanzenart Brassica oleracea besitzen einen verhältnismäßig hohen Gehalt an Glucosinolaten. Grünkohl enthält etwa 1 mg Glucosinolate pro Gramm Frischgewicht (Rouzaud G et al. Br J Nutr. 2003; 90(2):395-404). Über die Konzentration an Flavonoiden in Grünkohl sind recht unterschiedliche Mengen in der Literatur zu finden. Untersuchungen des Erfinders zeigen, dass die Trockenmasse von Grünkohl 1,5% bis 2% Flavonoide enthalten kann. Über die genauen Mengen der jeweiligen Glucosinolate bzw. Flavonoide ist relativ wenig bekannt.

Die vorliegende Erfindung erlaubt somit ausgehend von Brassica-Pflanzen die Herstellung von Zusammensetzungen mit einem hohen Anteil an aktiven Substanzen, die eine inhibierende Wirkung auf den Transporter SGLT-1 ausüben. Die erfindungsgemäß erhältlichen Extrakte können sowohl in trockener als auch in flüssiger Form hergestellt werden.

In einem weiteren Aspekt betrifft die vorliegende Erfindung die Verwendung eines wie oben beschriebenen Extrakts aus einer Pflanze der Gattung Brassica zur Herstellung einer pharmazeutischen Zusammensetzung oder eines diätetischen Lebensmittels oder eines Nahrungsergänzungsmittels zur Behandlung und/oder Prophylaxe einer hyperglykämischen Erkrankung. Bei der hyperglykämischen Erkrankung, deren Behandlung und/oder Prophylaxe durch die pharmazeutische Zusammensetzung oder durch das diätetische Lebensmittel oder Nahrungsergänzungsmittel angestrebt wird, handelt es sich vorzugsweise um Fettsucht, Diabetes oder eine von Diabetes verursachte Folgeerkrankung. Gemäß einer besonders bevorzugten Ausführungsform handelt es sich um Diabetes vom Typ II.

In einem noch weiteren Aspekt betrifft die vorliegende Erfindung eine pharmazeutische Zusammensetzung, die einen wie oben beschriebenen Extrakt aus einer Pflanze der Gattung Brassica umfaßt, zur Behandlung und/oder Prophylaxe einer hyperglykämischen Erkrankung. Die pharmazeutische Zusammensetzung ist insbesondere für die Behandlung und/oder Prophylaxe von Fettsucht, Diabetes (insbesondere Diabetes vom Typ II) oder eine von Diabetes verursachte Folgeerkrankung geeignet. Die pharmazeutische Zusammensetzung basiert vorzugsweise auf einem Extrakt aus den Blättern einer Brassica-Pflanze und enthält Flavonoide und/oder Glycoside der Pflanze. Besonders bevorzugt ist es, dass der Extrakt aus einer Brassica-Pflanze gewonnen wurde, die ausgewählt ist aus der Gruppe bestehend aus Grünkohl, Weißkohl, Brokkoli, Rotkohl, Wirsing und Chinakohl.

Die pharmazeutische Zusammensetzung kann mittels im Stand der Technik hinreichend bekannter Verfahren hergestellt werden. Derartige Verfahren sowie geeignete Hilfsstoffe und Träger werden beispielsweise in "Remington: The Science and Practice of Pharmacy", Lippincott Williams & Wilkins; 21. Auflage (2005) beschrieben. Die pharmazeutischen Zusammensetzungen können beispielsweise in Form von Granulaten, Pulvern, Tabletten, Kapseln, Sirup, Emulsionen, Suspensionen oder Lösungen vorliegen. Die Zusammensetzungen können für verschiedene Verabreichungsarten formuliert sein, beispielsweise für die orale, bukkale, sublinguale, transmukosale, rektale, subkutane, oder nasale Verabreichung. Die pharmazeutischen Zusammensetzungen können auch als Retardmittel formuliert werden.

Für die orale, bukkale und sublinguale Verabreichung werden in der Regel feste Formulierungen wie, z.B. Pulver, Suspensionen, Granulate, Tabletten, Pillen, Kapseln und Gelcaps verwendet. Diese können beispielsweise hergestellt werden, indem man die Extrakte mit mindestens einem Additiv oder mit mindestens einem Hilfsstoff vermischt. Derartige Hilfsstoffe und Träger werden beispielsweise in "Remington: The Science and Practice of Pharmacy", Lippincott Williams & Wilkins; 21. Auflage (2005) beschrieben. Als Additive oder Hilfsstoffe können beispielsweise mikrokristalline Cellulose, Methylcellulose, Hydroxypropylmethylcellulose, Casein, Albumin, Mannit, Dextran, Saccharose, Lactose, Sorbitol, Stärke, Agar, Alginate, Pectine, Kollagen, Glyceride oder Gelatine verwendet werden. Ferner können Zusammensetzungen für die orale Verabreichung Antioxidantien (z.B. Ascorbinsäure, Tocopherol oder Cystein), Gleitmittel (z.B. Magnesiumstearat), Konservierungsmittel (z.B. Paraben oder Sorbinsäure), Sprengmittel, Bindemittel, Verdicker, Geschmacksverbesserer, Farbstoffe und ähnliche Substanzen umfassen.

Flüssige Formulierungen der erfindungsgemäßen Extrakte, die sich für die orale Verabreichung eignen, können beispielsweise als Emulsion, Sirup, Suspension oder Lösungen vorliegen. Diese Formulierungen können unter Verwendung einer sterilen Flüssigkeit als Träger (z.B. Öl, Wasser, Alkohol oder Kombinationen derselben) in Form von flüssigen Suspensionen oder Lösungen hergestellt werden. Für die orale oder parenterale Verabreichung können pharmazeutisch geeignete Tenside, Suspensionsmittel, Öle oder Emulgatoren zugefügt werden. Für den Gebrauch in flüssigen Dosisformen geeignete Öle umfassen beispielsweise Olivenöl, Sesamöl, Erdnußöl, Rapsöl und Maisöl. Geeignete Alkohole umfassen Ethanol, Isopropylalkohol, Hexadecylalkohol, Glycerin und Propylenglykol. Suspensionen können ferner Fettsäureester, wie Ethyloleat, Isopropylmyristat, Fettsäureglyceride und acetylierte Fettsäureglyceride umfassen. Ferner werden Suspensionen häufig auch mit Substanzen wie Mineralöl oder Petrolatum versetzt.

Die vorliegende Erfindung betrifft ferner auch ein diätetisches Lebensmittel oder ein Nahrungsergänzungsmittel, das einen wie oben definierten Extrakt aus einer Pflanze der Gattung Brassica umfaßt, zur Behandlung und/oder Prophylaxe einer hyperglykämischen Erkrankung. Das diätetische Lebensmittel oder Nahrungsergänzungsmittel ist insbesondere für die Behandlung und/oder Prophylaxe von Fettsucht, Diabetes (insbesondere Diabetes vom Typ II) oder eine von Diabetes verursachte Folgeerkrankung geeignet. Vorzugsweise stammt der Extrakt aus einer Pflanze, die ausgewählt ist aus der Gruppe bestehend aus Grünkohl, Weißkohl, Brokkoli, Rotkohl, Wirsing und Chinakohl. Gemäß einer weiteren bevorzugten Ausführungsform wurde der Extrakt aus den Blättern der Brassica-Pflanze gewonnen und enthält Flavonoide und/oder Glycoside der Pflanze. Die diätetischen Lebensmittel und Nahrungsergänzungsmittel der vorliegenden Erfindung können darüber hinaus weitere nützliche Bestandteile, wie beispielsweise Ballaststoffe umfassen. Geeignete Ballaststoffe sind beispielsweise diejenigen, die sich aus dem Bockshornklee (Trigonella foenum-graecum) gewinnen lassen. Das diätetische Lebensmittel oder Nahrungsergänzungsmittel eignet sich in besonderer Weise für die Verwendung bei der Ernährung von Individuen, die an einer hyperglykämischen Erkrankung leiden oder bei denen das Risiko besteht, eine solche Erkrankung auszubilden. Die erfindungsgemäßen Lebensmittel oder Nahrungsergänzungsmittel können ferner im Rahmen der Kontrolle und/oder Bekämpfung von Übergewicht eingesetzt werden.

Die Erfindung wird nunmehr durch die nachfolgenden Beispiele veranschaulicht, wobei diese die Erfindung in keiner Weise beschränken.

### BEISPIELE

Die nachfolgenden Beispiele beschreiben die physiologische Wirkung des vorliegend beschriebenen Brassica-Extrakts:

### Beispiel 1

Es werden verschiedene Extrakte von Blättern von Brassica oleracea hergestellt.
- Ansatz (A):: 600 Gramm Blätter von Brassica oleracea convar. acephala var. sabellica (Grünkohl) wurden ge- friergetrocknet und anschließend pulverisiert. Es entstand ein Pulver (ca. 73 g), das zu glei- chen Teilen entweder in Wasser (Extrakt 1) oder in Ethanol (Extrakt 2) gelöst wurde. Die Extrak- te wurden vor der Prüfung auf Wirksamkeit auf Raumtemperatur (22°C bis 25°C) gebracht, und un- lösliche Bestandteile wurden abzentrifugiert.
- Ansatz (B):: 600 Gramm Blätter von Brassica oleracea convar. acephala var. sabellica (Grünkohl) wurden mit einem Cutter zerkleinert, und der pH-Wert der resultierenden Maische wurde mit Ascorbinsäure auf pH 5 eingestellt. Der enzymatische Aufschluß erfolgte mit den Enzymen Cellulase (0,025 ml Ve- gazym HC, 11000 Units/ml, Fa. Erbslöh) und Pek- tinase (0,025 ml Vegazym P, 40 Units/ml, Fa. Erbslöh) bei 37°C. Die Inkubationszeit betrug 4 Stunden. Anschließend wurden die unverdauten Pflanzenteile mit einem Sieb entfernt. Die nach- folgende Gefriertrocknung lieferte ein Pulver (ca. 75 g), das für die physiologischen Messun- gen in Wasser (Extrakt 3) oder Ethanol (Extrakt 4) gelöst wird.

Die biologischen Eigenschaften der Extrakte wurden mit Hilfe einer zellfreien elektrophysiologischen Messung geprüft, wie sie in der deutschen Patentanmeldung DE 10 2004 048397 beschrieben wird:

### Herstellung des SGLT1-Sensorchips

Für die Messungen wurden SLGT-1-Membranen aus einer CHO-Plasmamembran-Präparation verwendet (erhältlich bei IonGate Biosciences GmbH, Frankfurt am Main). 10 µl dieser Membran-Suspension (Proteinkonzentration ca. 2-3 mg/ml) wurden auf einem vorbehandelten Sensorchip mit einer runden Goldelektrode (3 mm Durchmesser, erhältlich von IonGate Biosciences GmbH, Frankfurt am Main) aufgetragen und über Nacht im Kühlschrank bei 4°C inkubiert.

### Aktivitätsmessung an SGLT1-exprimierenden Membranfragmenten

Die Durchflußzelle eines SURFE2R-Systems (IonGate Biosciences GmbH, Frankfurt am Main) mit integriertem, proteinbeladenem Sensorchip wurde zunächst mit Anlagerungspuffer (30mM Hepes, 5mM MgCl₂, 140 mM NaCl, 50 mM Mannitol, pH 7.0/N-Methyl-D-Glucamin, 0.2mM DTT) durchspült. Danach folgte die nichtaktivierende Lösung (140 mM NaCl, 2 mM MgCl₂, 30 mM Hepes pH 7.0/N-Methyl-D-Glucamin + 50 mM Mannitol). Mittels elektromechanischem 3/2-Wegeventil wurde anschließend auf aktivierende Lösung (140 mM NaCl, 2 mM MgCl₂, 30 mM Hepes pH 7/NMG + 5 mM oder 2,5 mM a-Methyl-D-Glucose (a-MDG)) umgestellt. Der Co-Transport von Natrium und Glucose führt zu einem positiven Signal, da positive Ladungsträger zur Membran verschoben werden.

### Aktivitätsmessung mit pflanzlichen Extrakten

Die Messungen wurden wie oben durchgeführt, allerdings wurde die Ruhelösung als auch die aktivierende Lösung mit unterschiedlichen Substratkonzentrationen (5 mM bzw. 2,5 mM a-MDG) versetzt. Die Signale wurden dann mit Kontrollmessungen (ohne pflanzlichen Extrakt aber gleiche Menge des jeweiligen Lösungsmittels) verglichen, um die physiologische Wirkung ermitteln zu können. Die inhibierende Wirkung der Extrakte auf SGLT-1 ist in Tabelle I zusammengefaßt.

**Tabelle 1: Hemmung der Aktivität des SGLT-1 in (%)**

| | Substratkonzentration: 5 mM a-MDG | Substratkonzentration: 2,5 mM a-MDG |
|---|---|---|
| Extrakt 1 | 15 % | 21 % |
| Extrakt 2 | 22 % | 39 % |
| Extrakt 3 | - | 51 % |
| Extrakt 4 | - | 58 % |

Diese Daten verweisen darauf, dass die vorliegenden physiologisch aktiven Extrakte eine inhibierende Wirkung auf SGLT-1 ausüben.

## Patentansprüche

1. Extrakt aus einer Pflanze der Gattung Brassica zur Behandlung und/oder Prophylaxe einer hyperglykämischen Erkrankung.

2. Extrakt nach Anspruch 1, wobei die Pflanze ausgewählt ist aus der Gruppe bestehend aus Grünkohl, Weißkohl, Brokkoli, Rotkohl, Wirsing und Chinakohl.

3. Extrakt nach den Ansprüchen 1-2, wobei der Extrakt aus den Blättern der Brassica-Pflanze gewonnen wurde.

4. Extrakt nach den Ansprüchen 1-3, wobei der Extrakt Flavonoide und/oder Glycoside der Pflanze enthält.

5. Extrakt nach den Ansprüchen 1-4, wobei die hyperglykämische Erkrankung Fettsucht, Diabetes oder eine von Diabetes verursachte Folgeerkrankung ist.

6. Extrakt nach Anspruch 5, wobei der Diabetes vom Typ II ist.

7. Verwendung eines Extrakts nach den Ansprüchen 1-4 zur Herstellung einer pharmazeutischen Zusammensetzung oder eines diätetischen Lebensmittels oder eines Nahrungsergänzungsmittels zur Behandlung und/oder Prophylaxe einer hyperglykämischen Erkrankung.

8. Verwendung nach Anspruch 7, wobei die hyperglykämische Erkrankung Fettsucht, Diabetes oder eine von Diabetes verursachte Folgeerkrankung ist.

9. Verwendung nach den Ansprüchen 7 oder 8, wobei der Diabetes vom Typ II ist.

10. Pharmazeutische Zusammensetzung, die einen Extrakt nach den Ansprüchen 1-4 umfasst, zur Behandlung und/oder Prophylaxe einer hyperglykämischen Erkrankung.

11. Pharmazeutische Zusammensetzung nach Anspruch 10, **dadurch gekennzeichnet, dass** die hyperglykämische Erkrankung Fettsucht, Diabetes oder eine von Diabetes verursachte Folgeerkrankung ist.

12. Pharmazeutische Zusammensetzung nach Anspruch 11, **dadurch gekennzeichnet, dass** der Diabetes vom Typ II ist.

13. Diätetisches Lebensmittel oder Nahrungsergänzungsmittel, das einen Extrakt nach den Ansprüchen 1-4 umfasst, zur Behandlung und/oder Prophylaxe einer hyperglykämischen Erkrankung.

14. Diätetisches Lebensmittel oder Nahrungsergänzungsmittel nach Anspruch 13, **dadurch gekennzeichnet, dass** die hyperglykämische Erkrankung Fettsucht, Diabetes oder eine von Diabetes verursachte Folgeerkrankung ist.

15. Diätetisches Lebensmittel oder Nahrungsergänzungsmittel nach Anspruch 14, **dadurch gekennzeichnet, dass** der Diabetes vom Typ II ist.
